# EUROPEAN PATENT APPLICATION

(11) **EP 2 740 532 A1**
(43) Date of publication of application: **11.06.2014**
(21) Application number: 12461563.4
(22) Date of filing: 18.12.2012
(51) Int. Cl.: B01J 20/30, A01N 25/28, A23L 1/22, A61K 47/00, B01J 13/02, C08B 30/12, C08L 3/00, A61K 9/50, C08B 30/14

(54) **Method of producing a starch-containing material**

(30) Priority: 06.12.2012 PL 40192712
(71) Applicant: Zachodniopomorski Uniwersytet Technologiczny w Szczecinie, 70-310 Szczecin (PL)
(72) Inventor: Bartkowiak, Artur, 71-667 Szczecin (PL); Ternovoy, Garri, 71-635 Szczecin (PL)
(74) Representative: Zawadzka, Renata

(57) **Abstract**

A method for the preparation of a starch material, according to the present invention, rely on the thermal treatment of the starch with the use of polar organic solvents, is characterized by that 5÷95% by weight of starch is mixed with an aqueous solution of polar organic solvents at a concentration of 1÷85% by weight, which is eventually a 0.01÷10 wt% emulsifier solution with HLB 8÷20 and is eventually mixed with 5÷95% by weight a 0.01÷10 wt% emulsifier solution with HLB 4÷20 in a non-polar solvent, to achieve 100% by weight of the starch mixture. The mixture is then subjected to the thermal treatment in the temperature range of 60÷220°C over a period of 1 second to 120 minutes with continuous stirring, subsequently the starch mixture is dried. After thermal treatment, the starch mixture may be subjected to texturizing relying on the addition of polar organic solvents or its aqueous solution at a temperature from -20 to +80°C in the concentration range of 30÷85% by weight to the starch mixture. The starch mixture is then concentrated followed by dehydration using lyophilisation or by washing on a filtration device by means of rinsing with polar organic solvent to obtain a 5÷50% by weight residue of polar solvent and/or its aqueous solution and is eventually dried.

## Description

The present invention relates to a method of manufacturing of porous starch sorbent.

The starch, and in particular its chemical derivatives are widely used in the field of immobilization of active substances in the food, cosmetics and paper industry. The microencapsulation process relaying on a spray drying of the emulsion systems on the basis of surface-active modified starch (starch octenylsuccinate, maltodextrin) belongs to the most frequently used. A phenomenon of undesirable microcapsules agglomeration quite often occurs and is caused by an inappropriate selection of the drying conditions or the composition of dried mixture, or the diffusion of immobilized substance proceeds outside the microcapsule. An alternative solution seems to be an increase of dry matter and the specific surface area through the application of porous food adsorbents. Unfortunately, a list of the adsorbents fulfilling the application conditions in the food is rather small and is restricted to the utilization of such mineral substances as activated carbon, silica, bentonite. In the majority of cases the food additives, medications or other substances, as immobilized active substances are used in a specified concentration in the final product, or in a powdered mixture or tablet. A desired dilution and a release profile of immobilized active substance is ensured through the application of various kinds of the filling substances in the form of organic matters, for example, the starch derivatives including hydroxypropyl methylcellulose, ethyl cellulose, or inorganic (aluminosilicates, silicon dioxide, titanium dioxide, calcium carbonate, activated carbon). In the case of using mentioned additives as the ingredients of food or medications, their usage is quite often is not economically justified or creates difficulty, because limitations in a permissible daily consumption may occur or the yield and the sensory properties of substrate are not appropriate for its target application. Several methods concerning the preparation of micro- and macro- porous starch grains have been described so far. In the description of the invention CN101240082(A) was disclosed a method for preparing the microporous starch grains, which relies on the addition of water in the amount of 20÷40% of their mass to the starch and the obtained mixture was left for a period from 24 to 48 hours, so that the water homogeneously impregnated the starch over its entire volume. The whole is then heated in the temperature range of 55÷90°C for 30÷120 minutes. The whole is refilled with water so that the weight ratio of starch and water amounted to 1: (2-4), is uniformly stirred, then amylase is added in the amount of 450÷650 ppm with the recalculation per gram of starch, followed by stirring for 2-6 hours at the pH in the range of 4.5÷5.5 and at a temperature of 35÷55°C. Subsequently, the starch is dried resulting in a microporous material which may be used as a substrate of medications, food additives, pesticides and fertilizers. A method for preparing a substrate from potato starch is known from a description of the invention CN101695477. The method comprises the following stages: mixing of potato starch with water and forming a starch dispersion with the concentration of 3.5÷5.0g/100ml and the pH 8÷11. The starch granules are then gellated at a constant temperature to obtain a starch gel. The third stage comprises the addition of edible oils and the emulsifier agent into a such system in order to prepare a reverse emulsion. The fourth stage comprises the addition of cross-linking agent, sodium bisulphate and ammonium persulfate. The reaction is carried out in the medium with the pH between 8÷11 and the obtained crosslinked gel is then washed, dried and ground. The prepared product is utilized as a substrate for extended-release medications. A method for preparing a biodegradable spherical porous starch as a substrate for insoluble medication is known from a description of the invention CN101880405. The process relies on a rapid stirring or homogenization of starch in an aqueous solution with methylbenzene and chloroform and the emulgation agent. The obtained dispersion is maintained at a temperature of-20°C. The whole is subjected to the gelation at temperature of 90÷150°C, dehydration, separation and the removal of methylbenzene/chloroform by means of anhydrous ethanol followed by the filtration process and filtrate drying in vacuum to obtain a biodegradable spherical porous starch. The prepared porous starch is suitable for the utilization as a substrate of hydrophobic medications. A method is known from the polish description of invention PL210309 for preparing a substrate with immobilized material, particularly with microorganisms, which penetrates into the porous structure of the substrate. The starch used as a carrier is humidified from 10 to 40% then is subjected to the extrusion with the compression ratio of screw auger from 1:1 to 3:1, screw auger revolutions from 60 to 200 rpm, feeder screw revolutions from 30 to 60 rpm, granulator knife revolutions from 250 to 500 rpm, nozzle diameter from 1 to 6 mm, temperature in the feeding zone from 320 to 350 K, temperature in the plastification zone from 330 to 370 K, temperature in the extrusion head zone from 340 to 375 K. Such prepared substrate is placed directly after the extrusion, in an aqueous dispersion containing an immobilized material and is left for a period of time from 2 to 48 hours. A description of the invention US3620842 provides a method for preparing a starch swelling in cold water by means of the thermal treatment under autogenous pressure at a temperature from 115 to 193°C in the aqueous solutions containing the polar organic solvents. A description of the invention US4634596 provides a method for preparing a cold water swelling fragmented starch. The method concerns a thermal treatment of the starch composition which is composed of at least two kinds of starch, wherein one starch component is essentially amylose free. A description of the invention US6261376 provides a method for preparing a thermally inhibited starch which is essentially free of undesired taste and flavour by dehydrating of previously pregelatinized starch with anhydrous polar solvents followed by a thermal treating of dry starch at a temperature of 140°C. A description of the invention US5547513 provides a method for preparing a pregelatinized starch with a high content of amylose by a complete dissolution of the starch grains at high temperature, filtering with diatomaceous earth, retrograding and spray drying of obtained dispersion. A description of the invention US7119195B2 provides a method for preparing a pharmaceutical starch by washing of waxed starch in the alkaline solutions followed by the dissolution and mechanical degradation of the mole mass of such starch. A description of the invention US4859377 discloses a method for the immobilization of the active components for insects control in a pregelatinized starch matrix, relying on a dispersion of the active component in a swelled starch matrix with the formation of coating by dissolved starch. A description of the invention US6147028 concerns a method for preparing the porous starch substrates by a enzymatic etching and a modification by siloxanes, capable for selective adsorbing of malodours.

A method of manufacturing a starch sorbent, in accordance with the present invention, rely on the thermal treatment of the starch with the use of polar organic solvents, is characterized by that a 5÷95 wt% of starch is blended with an aqueous solution of organic solvent at a concentration of 1÷85 wt%, which may be a 0.01÷10% emulsifier solution with the HLB value of 8÷20. Additionally, it may be added 5÷95 wt% of a 0.1÷10% emulsifier solution in a non-polar solvent with the HLB value of 4÷20. All the reactants are added in order to achieve 100 wt% of the starch mixture. This means that a reactant for a further treatment may have the four forms. In the first variant, the reactant is a starch with an aqueous solution of polar organic solvent. In the second variant, the reactant is a starch with an aqueous solution of polar organic solvent in which an emulsifier is additionally present with the HLB value of 8÷20. In a consecutive, the third variant, the reactant is a starch with an aqueous solution of polar organic solvent with emulsifier in the organic solvent with the HLB value of 8÷20. In the fourth variant, we have the reactant which is composed of the starch in an aqueous solution of polar organic solvent, in which is additionally present an emulsifier with the HLB value of 8÷20 and additionally occurs an emulsifier in an non-polar solvent with the HLB value of 4÷20. The aqueous solution of polar organic solvent, as well as the emulsifier, serves for controlled swelling of the starch grains and the extraction of amylose/amylopectin and to modify the sorption properties of the starch grains. Whereas the addition of emulsifier solution in a non-polar solvent facilitates the formation of the emulsion systems at a low content of the swelling agent - water/polar solvent aqueous solution, and additionally modifies the sorption properties of the starch granules. Subsequently, one of the fourth obtained reactants is subjected to the thermal treatment in the temperature range of 60÷220°C for 1 second ÷ 120 minutes with a continuous stirring. Moreover, the higher is a temperature the shorter is the time of thermal treatment. The prepared starch mixture is then dried. A starch mixture may be also subjected to the texturization relying on the addition of polar organic solvent or its aqueous solution at a temperature from -20 do +20 °C in the concentration range of 30÷85 wt% to the starch mixture. The texturization allows for the stabilization of the structure of starch granules due to shrinking (volume reduction) and a decrease of plastification of starch granules, what facilitates the concentration with reduced adhesion of starch granules. The starch mixture is then concentrated and dehydrated by the application of liophilization or by rinsing in the filtration device by means of a polar organic solvent to achieve a 5÷50 wt% residue of polar solvent and/or its aqueous solution. The product thus obtained can be dried. A porous starch sorbent is obtained.

As a starch may be used a granular starch and/or native and/or modified starch insoluble in cold water, a particularly maize starch with a low and/or intermediate and/or high content of amylose and/or potato starch and/or wheat starch and/rice starch and/or tapioca starch.

As a polar organic solvent is used a non-solvent of the starch, from the group of alcohols and/or with the aprotic properties including ketones and/or halogenoalkanes. From the group of alcohols is used particularly methanol and/or ethanol and/or 1-propanol and/or 1-butanol and/or isopropanol. From the group of ketones is used particularly acetone and/or butanone. Whereas from the group halogenoalkanes is used particularly dichloromethane. The polar organic solvents dissolving the starch may be used in a combination with the solvents which do not dissolve starch, that is in a mixture. In such a case the effect of starch dissolution will be limited by the application of the second solvent. Such polar organic solvents comprise glycerol and/or propanodiol and/or dimethylsufoxide and/or dimethylformamide and/or tetrahydrofuran and/or N-methylpyrrolidone and/or 2-aminoethanol and/or 1,4-dioxane and/or propylene carbonate and/or acetonitryle.

As a non-polar organic solvent are used the neutral solvents which do not swell the starch material including carbon dioxide and/or from the group of the aliphatic hydrocarbons and/or from the group of halogenoalkanes and/or from the group of the aromatic hydrocarbons and/or esters and/or ethers. As the aliphatic solvents are used particularly C4 ÷ C12 alkane hydrocarbons including butane and/or pentane and/or hexane and/or heptane and/or octane and/or paraffin oil and/or cycloalkanes including cyclopentane and/or cyclohexane. From the group of halogenoalkanes is used particularly chloroform and/or tetrafluoroethylene. From the group of the aromatic hydrocarbons as a solvent is used particularly xylene and/or toluene. From the group of esters is used particularly butyl acetate and/or ethyl acetate and/or the esters of fatty acids including animal fats and/or plant fats. As the solvents from the group of ethers is used particularly diethyl ether.

As an emulsifier with HLB 4÷ 20 are used the esters of sorbitan and/or fatty acids (Span) and/or the esters of polyoxyethylene sorbitan and/or fatty acids (Tween). Preferably sorbitan monooleate and/or sorbitan monostearate and/or sorbitan monooleate and/or sorbitan monooleate and/o sorbitan monooleate and/or monopalmitate sorbitan and/or monolaurate are used. The addition of emulsifier solution in a non-polar solvent allows to form a homogeneous mixture in which the starch is impregnated with the specified amount of water, what facilitates to perform a subsequent thermal treatment during a continuous stirring.

The thermal treatment in order to swell the starch is carried out by one of typical, well known methods. Preferably the thermal treatment is carried out in a reactor with heating jacket, stirrer and a condenser in the closed system or is carried out in the flow reactor. The thermal treatment is carried until achieving a maximum swelling of the starch without the disintegration of its granules when the extraction of amylose and/or amylopectin into a solution is not larger than 20% by weight. A higher concentration of polar organic solvent is used in the case of the lower values of the gelatinization temperature for a given starch, whereas a lower concentration of polar organic solvent solution is used for the starch with higher gelatinization temperatures.

Drying of the starch mixture is carried out by known method, preferably with the use of spray dryer, drum dryer, band dryer or fluidization dryer. Drying is performed in the temperature range of 60÷220°C to obtain the content of the remaining polar organic solvent and/or water in the amount of 0.1÷10% by weight in relation to the starch mass.

The starch can be modified by polyelectrolytes in order to form a polyelectrolyte complex with starch. A solution is added to the starch mixture of at least one polyelectrolyte with anionic and/or amphiphilic and/or cationic properties which is soluble in water and/or an aqueous solution of polar organic solvent. Each of added polyelectrolyte is used in the amount of 0.01÷10% by weight in relation to mass of the starch mixture. This means that a polyelectrolyte can be introduced prior and/or during and/or after each operations (of preparing the mixture of starch and solvent, texturization, concentration) with the exception of drying operations. The obtained starch sorbent may contain one polyelectrolyte or several polyelectrolytes added in one operation or in several operations. Preferably the polyelectrolyte is added to the starch mixture in the two operations:
- prior to and during the texturization,
- prior to and after the texturization,
- during and after the texturization.

Preferably in the second operations is used a polyelectrolytes with the properties (ionic character) opposite to that from the first operations, that is, for example, if at first (prior to or during) the first operations is added a polyelectrolyte with the anionic properties, then in the second operations (during or after) a polyelectrolyte with the cationic properties is added. This means that the polyelectrolytes are added pairwise: anionic - cationic, or anionic - amphiphilic, or cationic - anionic, or amphiphilic - anionic, or amphiphilic - cationic. This procedure allows for a change of kinetics of release of the immobilized substance and to improve the formation of protective coating (made of film-forming substance) around the starch granules during the microcapsulation. Preferably a pH value of the starch mixture after the addition of polyelectrolyte solution should be adjusted above the pI value of dissolved polyelectrolyte in the case of anionic polyelectrolyte or below the pI value in the case of cationic polyelectrolyte. Preferably the polyelectrolytes are added into the starch mixture prior the texturization so that their precipitation on the surface of granular starch could take place for the system with higher concentration of polar organic solvent.

The cross-linking of starch can be carried out in order to stiffen the swelled structure of starch granules. Preferably after the thermal treatment a starch mixture is crosslinked with the cross-linking agent in the amount of 0.1÷10% by weight in relation to the starch mass. A reaction medium pH is maintained in the pH range of 8÷12 whereas the cross-linking is carried out for 10 minutes÷48 hours in the temperature range of 20÷80°C. A non-toxic cross-linking agent is from the group of C2÷C6 dicarboxylic acids soluble in a polar organic solvent or an aqueous solution of polar organic solvent, particularly in the form of adipic acid. This agent may be also from the group of polyphosphates soluble in an aqueous solution of polar organic solvent, particularly in the form of sodium trimetaphosphate. Other cross-linking compounds regarded as the toxic compounds such as epichlorohydrin of phosphoryl chloride may be also used.

Preferably prior to and/or during the texturization and/or after the concentration and/or after drying is prepared a mixture of starch with the addition of non-volatile and inactive filling substance. The filling substance is added in the amount of 0.1÷30% by weight in relation to the starch mass. Such filling substance may have a hydrophilic character (with both ionic and nonionic properties) or a hydrophobic character. A hydrophilic filling substance is in the form of polar organic solvent solution or an aqueous solution of polar organic solvent. Whereas a hydrophobic filling substance is in the form of non-polar organic solvent solution and/or polar solvent and/or an aqueous solution of polar organic solvent. The filling substance is used in order to achieve optimum filling of pores of starch sorbent and to control the concentration and release of immobilized substance, added in further operations. The more filling substance is added, the smaller amount of immobilized substance could be immobilized in the starch pores. An inactive non-volatile filling substance can be used over the entire range of concentrations within the capacity limits of starch substrate. The addition of solution of hydrophobic filling substance has the purpose to increase the starch granules resistance against the rehydration and to improve the sorption capabilities towards the immobilized active substances after the drying process. Preferably, a neutral polar organic solvent non-swelling the starch material (carbon dioxide, butane, pentane, hexane, heptane, octane, cyclopentane, cyclohexane, diethyl ether) and/or polar solvent (methanol, ethanol, 1-propanol, isopropanol, 1-butanol) is used as an organic solvent of the filling substance.

After the concentration and eventual drying, to the swollen starch which may contain a filling substance, and/or polyelectrolytes and/or a cross-linking compound, can be added a hydrophilic immobilized substance. The hydrophilic immobilized substance is used in the form of an aqueous solution and/or polar organic solvent solution and/or in a mixture with the filling substance (this means that the filling substance may be also introduced to the starch together with the hydrophilic immobilized substance). The whole is then stirred until a consistence with the desired viscosity required in the drying process is achieved, subsequently is dried to obtain a loose starch sorbent impregnated by the immobilized substance. As a hydrophilic immobilized substance are used organic and/or inorganic acids and bases and/or their salts in the volume quantity smaller or equal to the specific capacity expressed in mL/g of used swollen mass of the starch sorbent. For example, as a hydrophilic immobilized substance are used typical organic food additives: sodium glutamate, potassium cyclamate and anaesthethic compounds: lidocaine hydrochloride, dextrometorfan sulphate and the like. After the concentration and eventual drying, to the starch sorbent which may contain a filling substance, and/or polyelectrolytes and/or a cross-linking compound, and/or a hydrophilic immobilized substance, is added a hydrophobic immobilized substance in a solution of a neutral polar organic solvent non-swelling the starch sorbent, and/or non-polar organic solvent and/or polar aprotic and/or polar protic and/or in the mixture with non-polar filling substance. The hydrophobic immobilized substance is used in the volume quantity smaller or equal to the specific capacity expressed in mL/g of used swollen mass of the starch sorbent. The whole is then stirred until a consistence with the desired viscosity required in the drying process is achieved, subsequently is dried to obtain a loose starch sorbent impregnated by the immobilized substance. As the hydrophobic (lipophilic - fat-soluble) immobilized substances are used, for example, unsaturated fatty acids and their esters, fat-soluble drugs, for example, vitamins A, D, E, K, hormones including sterols, pigments, flavour and taste substances including essential oils and other lipophilic active substances or their mixtures. As the hydrophobic immobilized substances are also used anetol tritionate, oridonin, kurkumin, vinpocetine, tacrolimus, berberine, nobiletyne, piroxicam, drugs against malaria beta-artemether and halofantrine, drugs against HIV UC 781, nimodypine, exemestane, antitumor drugs 9-nitrocamptothecin (9-NC), paclitaxel and seocalcitol, alprostadil, probucol, itraconazole, fenofibrate, acyclovir, symvastatin, xibornol, silymarin, alpha-asaron, enilkonazol, pueraryne, atorvastatin, heparin, carvedilol, ketoconazole, gentamicin, labrasol, flurbiprofen, celecoxib, danazol, cyclosporine and idebenone. Preferably, a neutral polar organic solvent non-swelling the starch material (carbon dioxide, aliphatic solvents, particularly butane, pentane, hexane, heptane, octane, cyclopentane, cyclohexane, the esters particularly butyl acetate, ethyl acetate, the ethers particularly diethyl ether, aromatic solvents particularly xylene, toluene, halogenoalkanes particularly chloroform, tetrafluoroethane) and/or a polar aprotic solvent (dichloromethane, ketones particularly acetone, butanone ) and/or a polar solvent (alcohols, particularly methanol, ethanol, 1-propanol, isopropanol, 1-butanol). The hydrophobic immobilized substance may be added to the starch material with previously added hydrophilic immobilized substance owing to which is obtained the starch substrate with immobilized substances with both hydrophilic and hydrophobic properties. The starch may also contain a previously added filling substance and/or combined with polyelectrolytes and/or be cross-linked.

The swollen starch and/or with filling substance and/or with each of immobilized substances and/or combined with polyelectrolytes and/or be cross-linked, can be covered by coating-forming substance. Preferably the starch mixture is prepared with the addition of 0.1÷30 wt% of hydrophilic film-forming substance with the ionic properties in the form of an aqueous solution or an aqueous solution of polar organic solvent or with the hydrophobic properties applied in the form of solution of polar organic solvent and/or non-polar solvent. The starch sorbent mixed in appropriate proportions with film-forming substance after coating the cellulosic surface, for example, paper or cardboard, followed by drying, enables the formation of functional coatings on the surface of packaging materials.

Preferably a mixture of starch sorbent and a solution containing a film-forming substance is subjected to microencapsulation and/or macroencapsulation. This allows to limit the oxidative degradation and to improve a controlled release of immobilized substance from the starch sorbent.

The microencapsulation relies on that, a starch sorbent is mixed with a solution of film-forming substance with a concentration from 0.1 to 30 wt% for 0.1÷15 minutes until a homogeneous dispersion is achieved. Subsequently, the sorbent is spray-dried at inlet air temperatures of 60÷220°C to enable the formation of a continuous shell around the starch sorbent impregnated by immobilized substance, preferably at temperatures of 120÷140°C. The microcapsulation enables the closure of each granule of the starch sorbent by the formation of stiffen coating from the film-forming substance. A thickness and the nature of a coating material of microcapsule will be decisive for the rate of release and the stability of immobilized substance. In association with this is created the possibility of dosage of the immobilized substance being in the solid or liquid state. This allows to achieve a controlled release of immobilized substance into the environment (atmosphere) and reaching during a specified time of a specific concentration profile of this substance in the target environment (mixture).

The macroencapsulation relies on that, a pre-granulation is used and/or tabletting a starch sorbent impregnated by immobilized substance and/or a microencapsulated starch sorbent with eventual formation of coating on the tablet (drageeing) with the utilization of solution of film-forming substance. Preferably, prior to the coating with film-forming substance, a pre-granulation is carried out of the starch sorbent impregnated by immobilized substance which is then subjected totabletting with the use of tablet press or preferably with rotary tablet press. Subsequently, a tabletted, impregnated with immobilized substance starch sorbent is coated with a solution of film-forming substance by encapsulation on a drum spreader at temperatures of 10÷80°C, preferably at temperatures of 30÷60°C. The macrocapsulation, being a tabletting process with drageeing, allows to form a tight, soluble in a controlled manner coating around a compressed, precisely determined dosage on a weight basis of the starch sorbent impregnated by immobilized substance and/or microcapsulated. The macrocapsulation allows to deliver the immobilized substance in the discrete form in precise dosages, what is suitable, for example, in the pharmaceutical applications.

As a film-forming substance are used the aqueous solutions of polymers with the anionic or amphihilic properties including proteins and/or carbohydrates, and/or sugars and/or poly(ethylene oxide) and/or polyvinylpyrrolidone. As the proteins are used gelatine and/or derivative of keratin including a sulfonated keratin, and/or albumin and/or zein, and/or soyabean protein, and/or casein and/or other proteins. As the carbohydrates are used particularly alginates and/or carrageenans and/or xanthan and/or gellan and/or pectin and/or guar gum and/or arabic gum and/or agar gum and/or cellulose sulphate and/or dextran sulphate and/or chondroitin sulphate and/or cellulose acetate phthalate, and/or carboxymethyl cellulose and/or hydroxypropyl methylcellulose and/or cellulose acetate succinate and/or anionic derivatives of the starch - octenylsuccinate and/or carboxymethyl starch and/or hydroxypropyl starch and/or starch hydrolysates. As the sugars are particularly used glucose and/or fructose and/or lactose and/or sucrose and/or xylitol and/or sorbitol and/or maltitol.

As the film-forming substances are used the aqueous solutions of polymers with the cationic properties, that is, chitosan and/or chitosan hydrolysates and/or chitosan derivatives and/or poly(dimethyl allylamine) chloride and/or DEAE dextran and/or the polymers containing the amine groups of different orders and/or ammonium salts and/or mixtures thereof and/or cationic derivatives of the starch and/or mixtures thereof.

Alternatively, as the film-forming substances are used the solutions of polymers soluble in the organic solvents with a non-polar character, particularly acids and/or salts of fatty acids, particularly magnesium stearate or calcium stearate or mixtures thereof with the esters of mono- and polyhydroxy alcohols and fatty acids particularly in the form of animal and plant waxes and fats and/or synthetic waxes, paraffin.

Another group constitute the film-forming substances soluble in the polar solvents, particularly polyethylene glycol and/or polyvinylpyrrolidone and/or polyvinyl alcohol and /or polyvinyl acetate and/or nitrocellulose and/or cellulose acetate and/or ethyl cellulose and/or polystyrene and/or poly(methyl methacrylate).

As a filling substance are used the solutions of polymers and/or the hydrophobic substances dissolved in the organic solvents. As the filling substances soluble in the non-polar organic solvents are used particularly plant and/or animal fats and/or oils and/or acids and/or salts of fatty acids particularly magnesium stearate and calcium stearate and/or paraffins and/or waxes and/or mixtures thereof.

As the filling substances soluble in the polar organic solvents are used particularly polyethylene glycol and/or polyvinylpyrrolidone and/or polyvinyl alcohol and /or polyvinyl acetate and/or nitrocellulose and/or cellulose acetate and/or ethyl cellulose and/or polystyrene and/or poly(methyl methacrylate).

Preferably as a polyelectrolyte with the anionic or amphihilic properties are used proteins and/or carbohydrates, and/or sugars and/or poly(ethylene oxide) and/or polyvinylpyrrolidone. As the proteins are used particularly gelatine and/or derivative of keratin including a sulfonated keratin, and/or albumin and/or zein, and/or soyabean protein, and/or casein and/or other proteins. As the carbohydrates are used particularly alginates and/or carrageenans and/or xanthan and/or gellan and/or pectin and/or guar gum and/or arabic gum and/or agar gum and/or cellulose sulphate and/or dextran sulphate and/or chondroitin sulphate and/or cellulose, that is carboxymethyl cellulose, hydroxypropyl methylcellulose, cellulose acetate phthalate, cellulose acetate succinate and/or anionic derivatives of the starch - octenylsuccinate and/or hydroxypropyl starch and/or starch hydrolysates. As the sugars are particularly used glucose and/or fructose and/or lactose and/or sucrose and/or xylitol and/or sorbitol and/or maltitol. Preferably as a polyelectrolyte with the cationic properties is used chitosan and/or chitosan hydrolysates and/or chitosan derivatives and/or poly(dimethyl allylamine) chloride and/or DEAE dextran and/or the polymers containing the amine groups of different orders and/or ammonium salts and/or mixtures thereof and/or cationic derivatives of the starch.

A solution according to the present invention is a method for manufacturing a porous sorbent on the basis of cheap and available unmodified starches, that has been designed as to be an efficient substrate of immobilized substances, particularly the hydrophobic substances. A proposed method for the manipulation of the structural changes of starch granules during gelatinization allows to produce on an industrial scale the novel food adsorbents with the extended surface area and high intrinsic capacity. The utilization of proposed sorbent as a replacement of currently used auxiliary substances enables, for example, the manufacture of additives to the food products with desired sensory characteristics features, increased efficiency and for the convenient application of adsorbed component, complete biodegradability and biocompatibility. The potential possibilities of industrial applications of such sorbents is the preparation of flavour-taste mixtures with defined organoleptic profiles or the application in the filler quality in the pharmaceutical drugs formulation or the performance of functional coatings for paper with the absorbing capacity or releasing the immobilized active substances or being the sensors towards specific substances. The advantage of solution is that it enables the manufacture of porous starch sorbent, which can be used in the nonimpregnated form as a sorbent, for example, of flavours, moisture, toxic substances and others. The method according to this invention enables the application of immobilized active substances with different properties and chemical character by the impregnation of porous starch sorbent. A starch material in the proposed solution is distinguished by biodegradability and biocompatibility, as well as by a wide spectrum of application in the fields of food technology, pharmaceuticals, protection of plant and animals. Another advantage of solution according to this invention is the protection on immobilized substances in the starch sorbent by means of coating on both micro and macro level, what enables the achievement of a longer storage time, easier and more convenient dosage of immobilized active substance and its release, indirectly controlled, in the target environment. A method according to the present invention for manufacturing the porous starch sorbent in the impregnated form allows to dilute the immobilized substance in the starch material and to modify its release profile by a change of coating solubility or the degree of rehydration of the starch granules. The coating of impregnated starch sorbent onto the cellulosic surfaces enables the manufacture of functional modifications of the cellulosic package surfaces, for example, the packages releasing flavours or absorbing specified groups of the chemical compounds or being the sensors of changes in the environmental conditions. A method according to the present invention allows to prepare in a short time a biodegradable and porous starch sorbent in a controlled and reproducible manner with the use of available equipment (reactor with a stirrer, flow reactor, centrifuge, spray dryer), without the need to utilize particularly toxic and/or carcinogenic chemicals (epichlorohydrin, phosphoryl chloride), with the utilization of organic solvents having a relatively low toxicity (methanol, ethanol, acetone, butane, pentane, hexane, carbon dioxide).

The preferred embodiments of the present invention are further presented in the following examples of performing thereof.

### Example I

A native maize starch with a high content of amylose in an amount of 20% by weight was mixed with 80% by weight of aqueous solution of polar organic solvent in the form of methanol with a concentration of 20% by weight. The mixture was subjected to thermal treatment at a temperature of 80°C for 120 minutes under continuous stirring. The thermal treatment was carried out in the reactor equipped with a heating jacket, stirrer and reflux condenser in the closed system, until the starch reached its maximum swelling without violation of integrity of its granules during the extraction of amylose to a solution at a level of 20% by weight. Subsequently, the texturization was carried out, that is, a polar organic solvent was added in the form of methanol at a temperature of 20°C in an amount of 40% by weight in relation to the starch. The starch mixture was then concentrated by washing out on a filtering unit by means polar organic solvent was added in the form of methanol to obtain 5% by weight residue of water and was dried in a spray dryer at a temperature of 60°C.

A starch material was obtained with the properties of sorbent that after drying absorbs odours, moisture or the hydrophilic substance solutions, the hydrophilic substance or solutions thereof, the polymer solutions in the organic solvents or aqueous.

### Example II

The starch was prepared according to the method of Example I, wherein instead of an aqueous solution of methanol was used a 5% by weight aqueous solution of propanol, which is a 0.5% polyoxyethylene monooleate emulsifier solution with HLB 15. The weight ratio of the propanol aqueous solution to the starch mass amounts to 45%. The mixture was then subjected to thermal treatment at a temperature of 80°C for 15 minutes. Subsequently, to the starch mixture mass was added in the amount of 50% by weight a propanol solution of vanilla oleoresin and a sodium caseinate solution in a 20% by weight propanol solution in water, when recalculated to the mass of used starch respectively, 5% by weight hydrophobic immobilized substance and 5% by weight film-forming substance. The starch mixture was spray-dried at an inlet air temperature of 120°C.

A microencapsulated vanilla flavour in the starch substrate was obtained in the amount of about 5% in relation to the mass of starch used.

### Example III

The starch was prepared according to the method of Example I, wherein instead of methanol was used ethanol as an aqueous solution with a concentration of 40% by weight. The weight ratio of the ethanol aqueous solution to the starch mass amounts to 30%. Additionally, the mixture was supplemented to 100 wt% by a 1 wt% solution of sorbitan monolaurate in a vegetable oil. The mixture was then subjected to thermal treatment at a temperature of 80°C for 15 minutes. Prior to the texturization, a 0.1 wt% chitosan aqueous solution was added in the ratio of 1:5 in relation to the starch mixture mass, and after the texturization was added 0.1 wt% sodium alginate aqueous solution in the ratio of 1:5 in relation to the starch mixture mass. After the concentration of solution to 30 % by weight in relation to starch mass, an oleoresin turmeric solution and zein in 60 wt% solution of ethanol in water was added in the amount of 50 % by weight in relation to starch mass, respectively 30 wt% hydrophilic immobilized active substance and 5 wt% film-forming substance when recalculated in relation to starch mass. The starch mixture was spray-dried at an inlet air temperature of 120°C.

A microencapsulated turmeric in the starch substrate was obtained in the amount of about 30% in relation to the mass of starch used.

### Example IV

The starch was prepared according to the method of Example I, wherein 40 wt% of maize starch with the amylose content of 25% was combined with 55 % by weight of ethanol aqueous solution at a concentration of 20 wt%, which is a 0.5% solution of polyoxyethylene sorbitan monooleate emulsifier with HLB 16.7. The mixture was supplemented to 100 wt% by a 0.5 wt% solution of sorbitan monolaurate in a vegetable oil with HLB 8.6.

Prior to the texturization, the starch was crosslinked with sodium trimetaphosphate in the amount of 10% in relation to starch mass for 10 minutes at a temperature of 80°C in a medium of at pH 8.5. After texturization, the starch mixture was concentrated using a filtration centrifuge. Subsequently, an aqueous solution of sodium glutamate and soyabean protein was added in the amount of 50% by weight, respectively 30 wt% hydrophilic immobilized active substance and 1 wt% film-forming substance when recalculated in relation to starch mass. The starch mixture was spray-dried at an inlet air temperature of 150°C.

A microencapsulated sodium glutamate in the starch substrate was obtained in the amount of about 30% in relation to the mass of starch used.

### Example V

A method was carried out according to Example I, wherein 80 % by weight of potato starch and 15% by weight of an aqueous solution of propanol and acetone mixed in the ratio 1:1 at a concentration of 40 wt% was used. Then a 0.1 wt% solution of emulsifier (sorbitan monooleate) in a non-polar solvent (heptane) with HLB 4.3 was added in the amount of 5% by weight. The thermal treatment was carried out at a temperature of 120°C for 0.5 minute in the flow reactor until achieving a maximum swelling of the starch without the disintegration of its granules when the extraction of amylopectin into a solution is not larger than 10% by weight. The texturization was carried out by adding an aqueous ethanol solution at a temperature of - 20°C and a concentration of 70 wt% in the amount of 50% by weight in relation to the starch mass. The concentration was carried out under spraying by anhydrous methanol using a filtration centrifuge to obtain 10% by weight residue of non-polar solvent and water.

A starch material having sorbent properties was obtained, which after eventual drying can absorb odours, moisture or solutions of hydrophilic substances, hydrophobic substances or solutions thereof, solutions of polymers in organic solvents or aqueous.

### Example VI

A method was carried out according to Example V, wherein 50% by weight of rice starch and tapioca starch mixed in the ratio 1:1 and 30% by weight of an aqueous solution of propanol at a concentration of 5 wt%. Then a 10 wt% solution of emulsifiers mixture with HLB 4.3 (30% by weight of sorbitan monolaurate with HLB 4.3 and 70% by weight of sorbitan monolaurate with HLB 6.7) in a non-polar solvent (paraffin oil) in the amount of 20% by weight. The thermal treatment was carried out at a temperature of 90°C for 1 minute in the flow reactor until achieving a maximum swelling of the starch without the disintegration of its granules when the extraction of amylopectin into a solution is not larger than 10% by weight. The texturization was carried out by adding a 50 wt% solution of acetone in ethanol solution in the amount of 50% by weight in relation to the starch mass at a temperature of 80°C. The concentration was carried out under spraying by acetone to obtain 50% by weight residue of - polar solvent and water. The starch mass in the obtained form can be used as a basis for the preparation of mixture with immobilized substance and/or filling substance before the actual drying or coating of the cellulosic surfaces.

### Example VII

The starch was prepared according to Example I, wherein after thermal treatment but prior to the texturization, a solution of polyelectrolytes with the anionic and amphihilic properties (sodium alginate and starch octenylsuccinate) dissolved in an aqueous solution of polar organic solvent (methanol) was added to the starch mass, wherein the polyelectrolytes mixed in the weight ratio of 1:2 were used in the amount of 0.1 % by weight in relation to the starch mass.

After texturization and the concentration to the methanol content of 20 wt%, a hydrophilic immobilized substance (20 wt% aqueous solution of sulphate dextromethorphan) was added to the starch mass in the amount of 60% by weight in relation to the starch mass, and was then mixed to a paste consistency followed by drying to obtain impregnated by immobilized substance a loose starch material containing 23 wt% of sulphate dextromethorphan.

The loose starch material impregnated by immobilized substance (sulphate dextromethorphan) was mixed for 5 minutes with a 1 wt% aqueous solution of film-forming substance (chitosan hydrolyzate) in the amount of 30% by weight to obtain a homogeneous dispersion.

The microcapsulation is carried out, that is a mixture of impregnated substrate with a solution of film-forming substance is spray-dried at an inlet air temperature of 220°C.

### Example VIII

The starch was prepared according to Example I, wherein after thermal treatment the starch mixture was crosslinked with the cross-linking in the form of adipic acid in the amount of 0.01 % by weight in relation to the starch mass. The reaction medium pH was maintained within pH 8, and the crosslinking was carried out for 48 hours at a temperature of 20°C.

A loose, impregnated by immobilized hydrophilic substance (5% ethanol solution of sodium glutamate) starch material (obtained after mixing the starch with a solution of immobilized substance in the proportion 40:60% by weight and drying) was mixed with a 0.5 wt% solution of filling substance (polydimethylallylamine chloride and DEAE dextran) in the amount of 10% by weight. Additionally, after the concentration and drying, to the starch material was added in the amount of 55 % by weight an immobilized hydrophobic substance in the form of solution of black pepper oleoresin in a non-polar organic solvent in the form of butane at a volume amount equal to the specific capacity in ml/g of used dry mass of the starch material. Subsequently, the content was mixed to obtain a paste consistency followed by drying to achieve a loose, impregnated by immobilized substances starch material containing flavours in the form of sodium glutamate and black pepper extract.

A microencapsulation is carried out which relies on that, an initial granulation was used and tabletting of impregnated starch material followed by drageeing with the utilization spraying of film-forming substance solution in the form of a 10 wt% solution of hydroxypropyl methylcellulose. The microencapsulation is performed by encapsulation in the drum coater at temperatures of 60÷70°C.

### Example IX

The starch was prepared according to Example I. A loose, impregnated by immobilized hydrophilic substance (20% ethanol solution of acetylsalicylic acid) starch material (obtained after mixing a solution of immobilized substance with the starch in the amount of 60% by weight and drying) was mixed with a 1.0 wt% aqueous solution of film-forming substance (starch octenylsuccinate) in the amount of 30% by weight in relation to the starch mass.

A microencapsulation is carried out which relies on that, an initial granulation was used and tabletting of impregnated starch material followed by encapsulation (drageeing) with the utilization spraying of film-forming substance solution in the form of a 10 wt% aqueous mixture including 4 wt% chitosan and 10 wt% poly(vinyl alcohol) and 4 wt% carnauba wax, 1 wt% polyoxyethylene-(20)-sorbitan monolaurate and 1 wt% citric acid. The microencapsulation is performed by encapsulation in the drum coater at temperatures of 60÷70°C.

### Example X

The starch was prepared according to Example I, wherein the starch concentration amounts to 30 % by weight in relation to the mixture mass and a 40 wt% aqueous solution of methanol was used. During the texturization a mixture of filling substances: 5 wt% nitrocellulose, and 1 wt% beeswax in methanol was added to the starch mixture, while the temperature was maintained within 70°C and the mixture was vigorously stirred. A spray-drying of the mixture was used at an inlet air temperature of 150°C.

A loose, impregnated by filling substance (nitrocellulose, beeswax) starch material was mixed with a hydrophobic substance being a 5 wt% solution of geraniol in butane in the amount of 10% by weight. Subsequently, the content was mixed to obtain a paste consistency followed by drying to achieve a loose, impregnated by immobilized hydrophobic substance starch sorbent material containing geraniol as a flavour.

An initial granulation and tabletting of impregnated starch material was carried out without the subsequent formation of protective coating by means of drageeing.

### Example XI

The starch was prepared according to Example I, wherein after thermal treatment (without texturization and concentration) to the starch mixture in a polar organic solvent acetone was added an ethanol solution of filling substance in the form of poly(ethylene oxide) in the amount of 30% by weight in relation to the starch mass. The prepared mixture was spray-dried at an inlet air temperature of 220°C for 1 second.

The obtained starch sorbent in the amount of 55% by weight was combined with a mixture of solution of hydrophobic immobilized substance from Example IV and non-volatile filling substance in the form of glycerol tristearate and a polar aprotic organic solvent acetone which were mixed together in the proportion of 10:20:70% by weight.

After evaporation of solvent was obtained the starch material with the content of about 10 wt% of the active component in the form of black pepper oleoresinoleoresin in relation to the mass of starch used.

### Example XII

The starch was prepared according to Example I (without texturization and concentration), wherein 20 % by weight native maize starch with the content of amylose 25 wt% was mixed with 80 % by weight an aqueous solution of a polar organic solvent in the form of ethanol with a 40 wt% concentration. The mixture was subjected to the thermal treatment at a temperature of 80°C for 5 minutes in the flow reactor. The starch mixture was then mixed with a 0.05 wt% aqueous solution of kappa-carrogeenan (anionic polyelectrolytes) in the ratio of 1:1 and was dried in the spray dryer at a temperature of 160°C.

The starch material was obtained with the sorbent properties, which was mixed in the ratio of 1:3 with a solution containing 2.5 wt% of black pepper oleoresinoleoresin as the hydrophobic immobilized substance and 7.5 wt% of lard as a filling substance in a hydrophobic solvent hexane and was spray dried at a temperature of 100°C under a nitrogen atmosphere:

### Example XIII

The product prepared according to Example XII was mixed in the ratio of 1:1 with a 0.1 wt% aqueous solution of cationic starch with the degree of substitution 0.05 (cationic polyelectrolyte). Lemon oil is used as a hydrophobic immobilized substance. The obtained mixture was coated onto a paper followed by drying on a belt dryer at a temperature of 160°C for 1 minute. The paper was obtained with a functional coating having fragrance release properties.

### Example XIV

A method of production similar to Example III, wherein instead of ethanol was used acetone in a mixture with starch. After thermal treatment was added a cross-linking agent in the form phosphoryl chloride in the amount of 2% by weight, the pH of the medium in the range 9. The stirring was carried out for 1 hour at a temperature of 30°C. After the concentration was added a filling substance in the form of 5% by weight ethanol solution of polyethylene glycol mixed at the ratio of 2:1.

### Example XV

A method similar to Example VII, wherein the macroencapsulation from Example IX was used after microencapsulation.

### Example XVI

The starch was prepared according to Example I, wherein instead of methanol was used ethanol, which was employed as an aqueous solution with the concentration of 40% by weight. The weight-ratio of aqueous ethanol solution to the starch mass amounts to 30%. Additionally, the mixture was supplemented to 100% with a solution of sorbitan monolaurate in vegetable oil. Additionally, a hydrophobic immobilized substance in the form of hydrocortisone in the amount of 2% by weight in relation to the starch was added to the mixture. The texturization and concentration was not used. The mixture was stirred at a temperature of 75°C for 20 minutes. Subsequently, a film-forming substance was added in the form of a 10 wt% aqueous dispersion of poly(vinyl acetate) in the amount of 3:1 solution to the starch. A spray-drying was used at a temperature of 140°C.

### Example XVII

The starch sorbent obtained according to Example I was charged with a film-forming substance in the form of poly(methyl methacrylate) in a non-polar solvent - toluene (5 wt% solution of PMM in toluene). The ratio of starch to film-forming substance amounts to 1:4. The obtained mixture was spray-dried at a temperature of 120°C and was then subjected to tabletting. A tabletted microencapsulated starch sorbent was obtained.

## Claims

1. A method for the preparation of a starch sorbent, according to the present invention, rely on the thermal treatment of starch with the use of polar organic solvents, **wherein** 5÷95% by weight of starch is mixed with an aqueous solution of polar organic solvents at a concentration of 1÷85% by weight, which is eventually a 0.01÷10 wt% emulsifier solution with HLB 8÷20 and is eventually mixed with 5÷95% by weight a 0.01÷10 wt% emulsifier solution with HLB 4÷20 in a non-polar solvent, to achieve 100% by weight of the starch mixture, then the mixture is subjected to the thermal treatment in the temperature range of 60÷220°C over a period of 1 second to 120 minutes with continuous stirring, subsequently the said starch mixture is dried or said starch mixture may be subjected to texturizing relying on the addition of polar organic solvents or its aqueous solution at a temperature from -20 to +80°C in the concentration range of 30÷85% by weight to the starch mixture, said starch mixture is then concentrated followed by dehydration using lyophilisation or by washing on a filtration device by means of rinsing with polar organic solvent to obtain a 5÷50% by weight residue of polar solvent and/or its aqueous solution and is eventually dried.

2. A method according to claim 1, **wherein** is used said granular starch and/or native and/or modified starch insoluble in cold water, particularly a maize starch with a low and/or medium and/or high content of amylose and/or potato starch and/or wheat starch and/or rice starch and/or tapioca starch.

3. A method according to claim 1, **wherein** as a polar organic solvent is used a solvent from the group of alcohols and/or with the aprotic properties including ketones and/or from the group of halogenoalkanes, particularly is used methanol and/or ethanol and/or 1-propanol and/or 1-butanol and/or propanodiol and/or isopropanol and/or acetone and/or butanone and/or glycerol butanone and/or propanodiol and/or dimethylsufoxide and/or dimethylformamide and/or tetrahydrofuran and/or N-methylpyrrolidone and/or 2-aminoethanol and/or 1,4-dioxane and/or propylene carbonate and/or acetonitryle.

4. A method according to claim 1, **wherein** as a non-polar organic solvent are used the neutral solvents which do not swell the starch including carbon dioxide and/or from the group of the aliphatic hydrocarbons and/or from the group of the aromatic hydrocarbons and/or from the group of halogenoalkanes and/or esters and/or ethers, particularly C4 ÷ C12 alkane hydrocarbons including butane and/or pentane and/or hexane and/or heptane and/or octane and/or chloroform and/or paraffin oil and/or cycloalkanes including cyclopentane and/or cyclohexane and/or xylene and/or toluene and/or chloroform and/or tetrafluoroethylene and/or butyl acetate and/or ethyl acetate and/or the esters of fatty acids including animal fats and/or plant fats and/or diethyl ether.

5. A method according to claim 1, **wherein** as an emulsifier with HLB 4÷ 20 are used the esters of sorbitan and/or fatty acids and/or the esters of polyoxyethylene sorbitan and/or fatty acids, particularly sorbitan monooleate and/or sorbitan monostearate and/or sorbitan monopalmitate and/or sorbitan monolaurate.

6. A method according to claim 1, **wherein** the thermal treatment is carried out in a reactor with heating jacket, stirrer and a condenser in the closed system or is carried out in the flow reactor until achieving a maximum swelling of the starch without the disintegration of its granules when the extraction of amylose and/or amylopectin into a solution is not larger than 20 % by weight, wherein a higher concentration of polar organic solvent is used in the case of the lower values of the gelatinization temperature for a given starch, whereas a lower concentration of polar organic solvent solution is used for the starch with higher gelatinization temperatures.

7. A method according to claim 1, **wherein** drying of the starch mixture is carried out with the use of spray dryer, drum dryer, band dryer or fluidized-bed dryer, in the temperature range of 60÷220°C to obtain the content of the remaining polar organic solvent and/or water in the amount of 0.1÷10 wt% in relation to the starch mass.

8. A method according to claim 1, **wherein** to the starch mixture is added a solution of at least one polyelectrolyte with anionic and/or amphiphilic and/or cationic properties which is soluble in water and/or an aqueous solution of polar organic solvent, wherein each of added polyelectrolyte is used in the amount of 0.01÷10% by weight in relation to mass of the starch mixture.

9. A method according to claim 1, **wherein** after thermal treatment the said starch mixture is crosslinked with the cross-linking agent in the amount of 0.1÷10% by weight in relation to the starch mass, whereas a reaction medium pH is maintained in the pH range of 8÷12 while the cross-linking is carried out for 10 minutes÷48 hours in the temperature range of 20÷80°C, and the cross-linking agent is from the group of C2÷C6 dicarboxylic acids soluble in a polar organic solvent or an aqueous solution of polar organic solvent, particularly in the form of adipic acid or is from the group of polyphosphates soluble in an aqueous solution of polar organic solvent, particularly in the form of sodium trimetaphosphate or is epichlorohydrin of phosphoryl chloride.

10. A method according to claim 1, **wherein** prior to and/or during the texturization and/or after the concentration and/or after drying is prepared a mixture of starch with the addition of non-volatile and inactive filling substance with the addition of hydrophilic or hydrophobic non-volatile and inactive filling substance in the amount of 0.1÷30% by weight in relation to the starch mass, whereas a hydrophilic filling substance is in the form of polar organic solvent solution or an aqueous solution of polar organic solvent, while a hydrophobic filling substance is in the form of non-polar organic solvent solution and/or polar solvent and/or an aqueous solution of polar organic solvent.

11. A method according to claim 1 or 8 or 9 or 10, **wherein** after the concentration and eventually drying is formed a mixture of the starch sorbent with the addition of hydrophilic immobilized substance in the form of an aqueous solution and/or a polar organic solvent solution and/or in the mixture with filling substance and the mixture is then stirred until a consistence with the desired viscosity required in the drying process is achieved, subsequently is dried to obtain a loose starch sorbent impregnated by the immobilized substance, wherein as a hydrophilic immobilized substance are used organic and/or inorganic acids and bases and/or their salts in the volume quantity smaller or equal to the specific capacity expressed in mL/g of used swollen mass of the starch sorbent.

12. A method according to claim 1 or 8 or 9 or 10 or 11, **wherein** after the concentration and eventually drying is formed a mixture of the starch sorbent with the addition of a hydrophobic immobilized substance in a solution of a neutral and non-swelling the starch non-polar organic solvent and/or polar aprotic and/or polar protic and/or in the mixture with non-polar filling substance, whereas the hydrophobic immobilized substance is used in the volume quantity smaller or equal to the specific capacity expressed in mL/g of used dry mass of the starch sorbent, and said mixture is then stirred until a consistence with the desired viscosity required in the drying process is achieved, subsequently is dried to obtain a loose starch sorbent impregnated by the immobilized substance.

13. A method according to claim 1 or 8 or 9 or 10 or 11 or 12, **wherein** the starch mixture is prepared with the addition 0.1÷30% by weight 15 of film-forming substance, whereas the film-forming substance with the hydrophilic properties is used in the form of an aqueous solution and/or a polar organic solvent solution, while with the hydrophobic properties is used in the form of solution of polar organic solvent and/or non-polar.

14. A method according to claim 13, **wherein** a mixture of the starch sorbent and a solution of film-forming substance is subjected to microencapsulation relying on spray drying or fluidized drying and/or is subjected to macroencapsulation relying on the granulation and/or tabletting of the starch sorbent with immobilized substance followed by eventual applying a coating to the tablet.

15. A method according to claim 13, **wherein** as a film-forming substance are used the aqueous solutions of polymers with the anionic or amphihilic properties including gelatine and/or derivative of keratin including a sulfonated keratin, and/or albumin and/or zein, and/or soyabean protein, and/or casein and/or other proteins and/or alginates and/or carrageenans and/or xanthan and/or gellan and/or pectin and/or guar gum and/or arabic gum and/or agar gum and/or cellulose sulphate and/or dextran sulphate and/or chondroitin sulphate, and/or cellulose derivatives including carboxymethyl cellulose, hydroxypropyl methylcellulose, cellulose acetate phthalate, cellulose acetate succinate, and/or anionic derivatives of the starch including starch octenylsuccinate and/or carboxymethyl starch and/or hydroxypropyl starch and/or starch hydrolysates, and/or polyethylene glycol and/or polyvinylpyrrolidone and/or glucose and/or fructose and/or lactose and/or sucrose and/or xylitol and/or sorbitol and/or maltitol.

16. A method according to claim 13, **wherein** as a film-forming substance are used the aqueous solutions of polymers with the cationic properties including chitosan and/or chitosan hydrolysates and/or chitosan derivatives and/or poly(dimethyl allylamine) chloride and/or DEAE dextran and/or the polymers containing the amine groups of different orders and/or ammonium salts and/or mixtures thereof and/or cationic derivatives of the starch and/or mixtures thereof.

17. A method according to claim 13, **wherein** as a film-forming substance are used the solutions of polymers soluble in the organic solvents with a non-polar character, particularly acids and/or salts of fatty acids particularly magnesium stearate or calcium stearate or the mixtures thereof with the esters of mono- and polyhydroxy alcohols and fatty acids particularly-in the form of animal and plant fats or waxes and/or aliphatic hydrocarbons particularly in the form of synthetic waxes, paraffin.

18. A method according to claim 13, **wherein** as a film-forming substance are used the solutions of polymers soluble in the polar and non-polar solvents particularly polyethylene glycol and/or polyvinylpyrrolidone and/or polyvinyl alcohol and /or polyvinyl acetate and/or nitrocellulose and/or cellulose acetate and/or ethyl cellulose and/or polystyrene and/or poly(methyl methacrylate).

19. A method according to claim 10, **wherein** as a filling substance are used the solutions of polymers and/or hydrophobic substances in the non-polar organic solvents, particularly are used the esters of mono- and polyhydroxy alcohols and fatty acids particularly in the form of animal and plant fats or waxes and/or aliphatic hydrocarbons particularly in the form of synthetic waxes, paraffin.

20. A method according to claim 10, **wherein** as a filling substance are used the solutions of polymers and/or hydrophobic substances in the polar organic solvents, particularly are used polyethylene glycol and/or polyvinylpyrrolidone and/or polyvinyl alcohol and /or polyvinyl acetate and/or nitrocellulose and/or cellulose acetate and/or ethyl cellulose and/or polystyrene and/or poly(methyl methacrylate).

21. A method according to claim 8, **wherein** as a polyelectrolyte with the anionic or amphiphilic properties are used gelatine and/or derivative of keratin including a sulfonated keratin, and/or albumin and/or zein, and/or soyabean protein, and/or casein and/or other proteins and/or alginates and/or carrageenans and/or xanthan and/or gellan and/or pectin and/or guar gum and/or arabic gum and/or agar gum and/or cellulose sulphate and/or dextran sulphate and/or chondroitin sulphate, and/or cellulose derivatives including carboxymethyl cellulose, hydroxypropyl methylcellulose, cellulose acetate phthalate, cellulose acetate succinate, and/or anionic derivatives of the starch including starch octenylsuccinate and/or carboxymethyl starch and/or hydroxypropyl starch and/or starch hydrolysates, and/or polyethylene glycol and/or polyvinylpyrrolidone and/or glucose and/or fructose and/or lactose and/or sucrose and/or xylitol and/or sorbitol and/or maltitol.

22. A method according to claim 8, **wherein** as a polyelectrolyte with the cationic properties are used chitosan and/or chitosan hydrolysates and/or chitosan derivatives and/or poly(dimethyl allylamine) chloride and/or DEAE dextran and/or the polymers containing the amine groups of different orders and/or ammonium salts and/or mixtures thereof and/or cationic derivatives of the starch and/or mixtures thereof.
